# NOUVEAU FASCICULE DE BREVET EUROPEEN

(11) **EP 0 906 248 B2**
(45) Date de publication et mention de la décision concernant l'opposition: **12.11.2003**
(45) Mention de la délivrance du brevet: 24.01.2001
(21) Numéro de dépôt: 97909392.9
(22) Date de dépôt: 13.10.1997
(51) Int. Cl.: C02F 11/12, C02F 11/04

(54) **PROCEDE DE TRAITEMENT ET VALORISATION ENERGETIQUE OPTIMISEE DES BOUES D'EPURATION URBAINE ET INDUSTRIELLE**
OPTIMIERTE METHODE ZUR BEHANDLUNG UND ENERGIELIEFERTEN VERWERTUNG VON KOMMUNALEM UND INDUSTRIELLEM KLÄRSCHLAMM
OPTIMISED METHOD FOR THE TREATMENT AND ENERGETIC UPGRADING OF URBAN AND INDUSTRIAL SLUDGE PURIFYING PLANTS

(30) Priorité: 06.01.1997 FR 9700050
(43) Date de publication de la demande: 07.04.1999
(73) Titulaire: Bouchalat, Youssef, 74500 Evian-les-Bains (FR)
(72) Inventeur: Bouchalat, Youssef, 74500 Evian-les-Bains (FR)
(86) Numéro de dépôt international: FR9701819
(87) Numéro de publication internationale: WO98030506

(56) Documents cités:
- EP-A- 0 564 298
- DE-A- 4 138 036
- DE-A- 19 502 856
- DE-C- 3 842 446
- FR-A- 2 240 890
- ATV-Handbuch: Klärschlamm, Verlag Ernst und Sohn, 4.AQuflage1996
- Hamburger Klärschlamm-Bewässerung-und Trocknungsanlage in Betrieb aus awt.Abwassertechnik Heft 5/1992
- Buss-schlammtrocknung Beitrag aus dem Seminar "Klärschlammentsorgung", VDI-Bildungswerk, Düsseldorf 1989

## Description

La présente invention concerne un procédé de traitement et de valorisation énergétique des boues produites dans les stations d'épuration des eaux usées d'origine urbaine et industrielle.

Le procédé objet de la revendication 1 est caractérisé par une combinaison inédite de moyens connus et expérimentés séparément, permettant de « libérer » l'énergie contenue dans les boues d'épuration et d'obtenir un bilan énergétique positif, égal à ≅ 5,7 tonnes équivalent pétrole / an / 1000 équivalents habitants.

Le procédé objet de la revendication 1 traite complètement le problème posé par l'élimination des boues d'épuration sans transfert de nuisances, en protégeant les sols, les nappes et l'environnement contre la pollution bactériologique et olfactive et celle des métaux lourds ; il est caractérisé en ce que :
1 ) Son coût d'investissement est compétitif et s'élève à environ 10 % de celui, pour le même nombre d'équivalents habitants, d'une usine d'incinération d'ordures ménagères avec récupération de chaleur et épuration conforme des fumées.
2 ) Son automatisme est suffisamment poussé avec toutes les sécurités pour permettre de l'exploiter en automatique sans présence de personnel, sachant qu'il n'utilise pas de vapeur sous pression.
3 ) Son entretien est simple et peu coûteux ; il nécessite l'intervention soit du personnel d'entretien de la station d'épuration, soit d'une société extérieure de maintenance.
4 ) Son coût global de traitement complet de la tonne de boue est tout à fait compétitif en comparaison au coût d'une élimination conforme des boues d'épuration.

Il en résulte une incitation économique à créer des nouveaux investissements liés au procédé objet de l'invention, qui sont ainsi rentables et créateurs d'un nombre important d'emplois stables induits par la construction des équipements chez les fournisseurs.

Le problème posé par les boues d'épuration préoccupe aujourd'hui sérieusement les Collectivités locales et l'Etat, étant donné leur tonnage considérable qui ne cesse d'augmenter, et les risques de pollution toxique, bactériologique et olfactive qu'elles induisent pour les sols, les nappes et l'environnement. Il est donc urgent de disposer d'une technique optimale de traitement et de valorisation de ces boues qui soit sûre, durable, écologique, économique et applicable à tous les cas ; c'est ce que propose le procédé objet de l'invention, qui s'applique à toutes les stations d'épuration de capacité supérieure ou égale à 30.000 équivalents habitants.

La destination actuelle des boues d'épuration est, après leur déshydratation mécanique, soit l'épandage agricole, soit la mise en décharge, et parfois leur incinération conjointe, avec ou sans séchage thermique préalable, avec les ordures ménagères. Cette dernière destination est onéreuse en investissement et n'est pas optimisée en valorisation énergétique des boues.

La mise en décharge des boues d'épuration est appelée à disparaître du fait de son interdiction annoncée pour 2002.

L'épandage agricole des boues d'épuration, de plus en plus pratiqué actuellement, est caractérisé par le fait que la forte teneur des boues en P205 et le relativement faible besoin des plantes en engrais phosphorique inscrivent le phosphore comme élément limitant et identifient les boues à un engrais « retard » libérant lentement les éléments fertilisants.

Il y a un risque de transmission des maladies pour l'homme et l'animal par les boues d'épuration épandues sur les champs ou en décharge ; en Suisse par exemple le risque de maladie est réduit car les boues épandues sur des terres agricoles doivent toujours être « hygiénisées » et ne doivent pas contenir plus de 100 entérobactériacées par gramme et aucun oeuf de ver contagieux. Une telle obligation n'existe pas en France.

Même après une hygiénisation, l'utilisation des boues comme engrais phosphorique retard doit être effectuée avec « parcimonie » à cause de leur apport en métaux lourds, pour éviter à très long terme (voir tableau ci-après) d'entraîner une saturation des sols en métaux lourds ; en effet le délai en années de saturation des sols en métaux lourds provenant d'un épandage de boues à raison de 2,5 t MS / ha / an, selon les normes fixées par l'ordonnance suisse sur la protection des sols, publié dans les « Nouvelles de l'EAWAG n° 28 de septembre 1989 », s'établit comme suit pour deux qualités de boues :

| Métal | Teneur moyenne mg / kg MS | Délai en années | Faible teneur mg / kg MS | Délai en années |
|---|---|---|---|---|
| Zinc | 1500 | 150 | 100 | 240 |
| Cuivre | 800 | 80 | 250 | 240 |
| Cadmium | 5 | 180 | 1 | 900 |
| Mercure | 5 | 160 | 1 | 800 |

La pratique du « remplissage » du sol jusqu'aux normes du fait de la généralisation progressive de l'épandage agricole des boues d'épuration, n'est pas conforme à une bonne gestion du patrimoine naturel de l'humanité; la prudence doit donc être de mise car il faut admettre que l'assainissement des sols pollués serait une tâche fastidieuse et très coûteuse pour les générations futures. Par ailleurs l'interdiction de la mise en décharge des boues d'épuration à brève échéance ( 2002 ), est à cet égard très sécurisante.

Concernant les toxiques organiques présents également dans les boues d'épuration, à savoir : LAS (alkyl-benzène sulfonate linéaire), NP (nonylphénol), PAK (hydrocarbures aromatiques polycycliques), Sn-OC (composés organo-étains), PCB (polychlorobiphényles), HCB (héxachlorobenzène) et LI (lindane), il manque selon l'information des « Nouvelles de l'EAWAG, septembre 89 » une meilleure évaluation des risques encourus dans l'utilisation agricole des boues d'épuration.

Le procédé objet de la présente invention, permet de remédier à ces inconvénients ; il prévoit la « valorisation énergétique » des boues préalablement séchées thermiquement jusqu'à 92 % de matières sèches, comme « combustible d'appoint » aisément stockable et dosable automatiquement dans les fours d'usines d'incinération des ordures ménagères, équipées de récupération d'énergie et d'installation d'épuration des fumées séparant les métaux lourds, qui subissent un traitement conforme à la protection des sols et des nappes.

Le tonnage de ces boues séchées représente en moyenne, en France, « 4,5% » de celui des ordures ménagères pour un même nombre d'équivalents habitants. Leur emploi comme combustible d'appoint permettra de combler les « creux » de charge thermique des fours-chaudières dus à la variabilité du taux d'humidité des ordures ménagères et de leur composition, donc de leur PCI (pouvoir calorifique inférieur), et d'assurer une production de vapeur « constante » et égale dans la mesure du possible, à leur capacité nominale, grâce aux équipements de stockage adéquat et de dosage automatique des boues séchées qui font partie du procédé de l'invention, et qui seront installés sur le site de l'usine d'incinération des ordures ménagères le plus proche de celui de la station d'épuration.

Les dessins annexés illustrent le « procédé » objet de l'invention :

La figure 1 représente les équipements relatifs aux cinq premières fonctions du procédé.

La figure 2 représente la sixième fonction du procédé. La figure 3 représente une « variante » de réalisation des fonctions 1 et 2 du procédé.

La figure 4 représente l'application du procédé au traitement complet du lisier et sa transformation en engrais granulé.

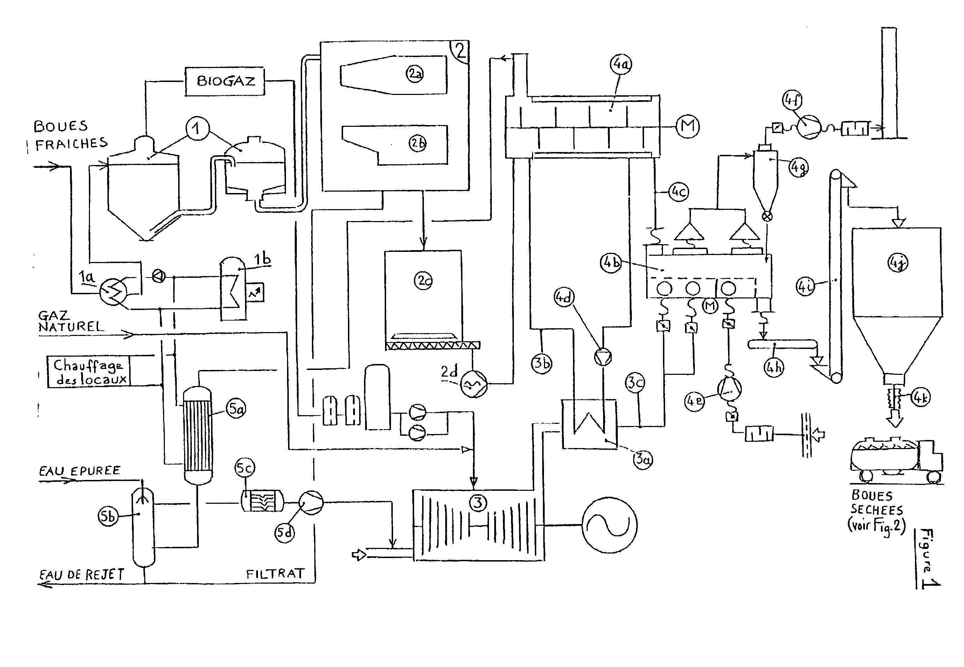

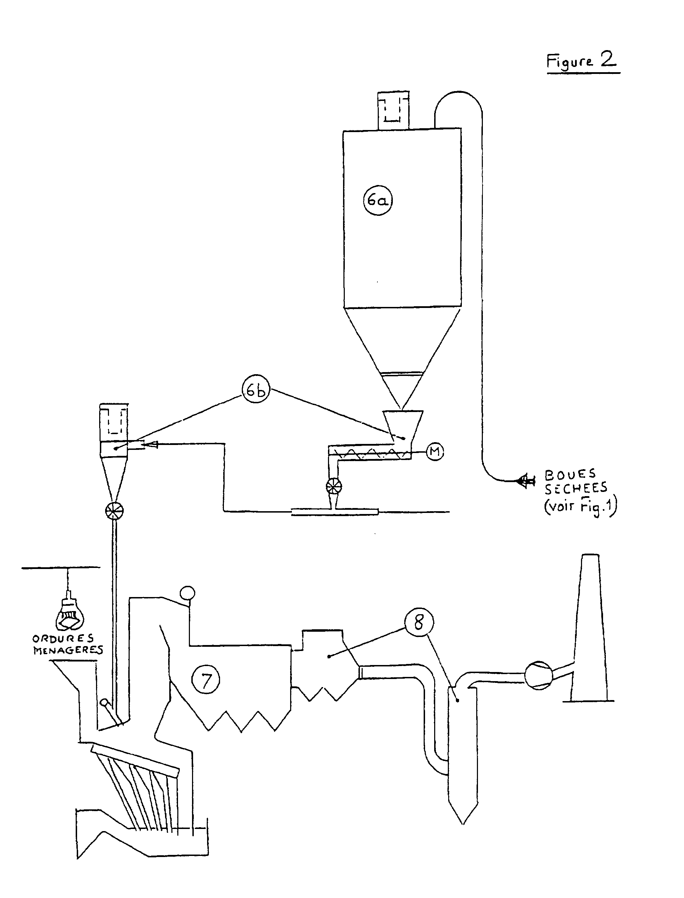

En référence aux figures 1 et 2 représentant le schéma de procédé, les fonctions 1 à 5 groupées pour la première fois sur le site d'une station d'épuration, et la fonction 6 installée pour la première fois sur le site d'une usine d'incinération, sont décrites ci-après :
1 ) La digestion anaérobie (1) des boues fraîches liquides qui « libère » une partie de l'énergie des boues sous forme de biogaz. Celui-ci après traitement par dessication et filtration, est stocké en vue de son utilisation. Le PCI du biogaz s'élève à environ 5500 Kcal/Nm3 soit 6,395 kwh/Nm3. Dans le procédé selon l'invention, la digestion anaérobie s'effectue à la température de 35 °C à l'abri de l'air pendant environ 20 jours dans un réacteur conventionnel unique. Le chauffage des boues fraîches liquides est réalisé à travers l'échangeur de chaleur (1a) utilisant l'eau chaude produite grâce à la chaleur de condensation des buées de séchage. Pour le premier démarrage de la digestion anaérobie une petite chaudière électrique (1b) assurera à l'aide de l'échangeur de chaleur (1a) le premier chauffage des boues.
   Selon des modes particuliers de réalisation, la digestion anaérobie conventionnelle peut être remplacée dans le procédé selon l'invention, par une digestion anaérobie avec séparation des deux phases d'hydrolyse acidogénèse et de méthanisation, ou par une stabilisation aérobie thermophile suivie d'une digestion anaérobie.
2 ) La déshydratation mécanique (2) des boues digérées jusqu'à 22 à 24 % de matières sèches à l'aide d'une centrifugeuse (2a), d'un filtre à bande pressante (2b) ou tout autre dispositif de déshydratation mécanique sans limitation du degré de siccité obtenue. La déshydratation mécanique des boues nécessite généralement l'adjonction de polymères pour la floculation préalable des boues liquides, permettant d'obtenir pour une siccité optimale des boues déshydratées, une meilleure qualité du filtrat qui retourne en tête de la station d'épuration. Les boues déshydratées sont amenées dans un silo de stockage tampon (2c), d'où elles sont dosées à l'aide d'une pompe spéciale (2d) vers le séchage thermique. Le rôle du silo tampon est de pouvoir faire fonctionner le séchage thermique en « continu » alors que la déshydratation mécanique peut fonctionner en discontinu.
3 ) La combustion du biogaz enrichi avec un faible appoint de gaz naturel, dans une « turbine à gaz (3) » dont l'application, objet de la revendication 2, est caractérisée en ce que l'installation de cogénération fonctionne « en énergie totale », entraînant un accroissement de son rendement global supérieur à 16 % par rapport à celui d'une cogénération classique, grâce à sa combinaison « judicieuse » avec un système de séchage thermique des boues à deux appareils, dont le premier utilise (circuit 3b) « les 100 % » de l'énergie récupérée sur les gaz chauds dans la chaudière de récupération (3a), et le deuxième emploie (circuit 3c) comme fluide de chauffe « les 100 % » du débit des gaz chauds à la température de sortie de la chaudière de récupération (3a), directement sans aucune adaptation ni équipement spécial complémentaire.
   Selon des modes particuliers de réalisation, la turbine à gaz peut être remplacée dans le procédé selon l'invention, par un moteur à gaz ou tout autre système de cogénération, et le combustible peut être remplacé dans le procédé selon l'invention, par du biogaz seul ou du gaz naturel seul.
4) Le séchage thermique (4) des boues déshydratées mécaniquement jusqu'à 92 % de matières sèches et en même temps le refroidissement des boues séchées, à l'aide d'un système comprenant deux appareils en série, directement reliés entre eux par une goulotte (4c), « sans » équipement de mélange en amont de boues séchées recyclées et de boues humides, donc « sans » recyclage de boues séchées et « sans » équipement de mélange et granulation intermédiaire ; entraînant ainsi une simplification considérable de l'installation de « séchage et refroidissement » des boues.
   La description de ces deux appareils, performants, qui sont retenus dans le procédé selon l'invention, pour le séchage et le refroidissement des boues est comme suit :
   - Le premier appareil (4a) est un « sécheur rotatif à couche mince » du type indirect, à double enveloppe chauffée par circulation d'huile thermique elle-même réchauffée dans la chaudière de récupération (3a) susvisée grâce à la pompe de circulation (4d).

   Selon des modes particuliers de réalisation, la chaudière de récupération (3a) peut produire dans le procédé selon l'invention, de la vapeur saturée sous pression, qui sera utilisée comme fluide de chauffe dans la double enveloppe du sécheur à couche mince à la place de l'huile thermique.
   L'échange de chaleur s'effectue à travers l'enveloppe interne sur laquelle sont étalées en couche mince les boues à sécher. Le coefficient de transfert de chaleur est élevé du fait de la faible épaisseur de la couche mince de boue, dont le renouvellement en contact avec la paroi est intense grâce à la forte turbulence de la couche mince créée par une vitesse linéaire des extrémités des pales voisine de 8 m/s.
   Le sécheur à couche mince est un sécheur éprouvé qui permet de sécher des boues d'épuration avec des siccités variables à l'entrée, sans recyclage de boues séchées, en fournissant à la sortie des boues à la siccité voulue sans craindre la phase extrêmement visqueuse et collante, dite phase plastique, que traversent les boues d'épuration lors du séchage thermique au-delà d'une siccité voisine de 50 % MS (% de matières sèches), laquelle limite est variable en fonction de la nature des boues.
   Le rotor du sécheur est équipé de pales réglables et amovibles qui sont à l'origine de la grande flexibilité du sécheur, et assurent l'étalement des boues en couche mince et son avancement continu jusqu'à la sortie du sécheur à environ 64 % de matières sèches, c'est-à-dire au-delà de la phase plastique des boues, sous forme granulée grâce au dispositif interne de granulation du sécheur.
   Au cours du séchage s'effectue d'abord le chauffage des boues entrantes jusqu'à la température de 100 °C environ, puis s'effectue à cette température l'évaporation de l'eau contenue dans les boues ; du fait de l'élévation de leur température à 100 °C au cours du séchage, les boues sont hygiénisées et leur pollution bactériologique est supprimée en quasi totalité.
   L'évaporation de l'eau contenue dans les boues se poursuit dans le deuxième appareil de séchage (voir ci-après) jusqu'à une siccité de 92 % MS environ avec obtention de granulés stables exempts de poussières ; du fait de la réduction de l'humidité des boues à la fin du séchage, à une valeur bien en-dessous des 15 % d'eau, le risque de reprise de la fermentation des boues séchées avec formation de mauvaises odeurs est éliminé.
   - Le deuxième appareil (4b) est un sécheur/ refroidisseur vibré à lit fluidisé qui assure la fin du séchage des boues de 64 % MS jusqu'à 92 % MS environ par convection au moyen des gaz chauds sortie chaudière de récupération (3a) traversant une sole fixe perforée.

   Les boues préséchées à environ 64 % MS et à la température d'environ 100 °C tombent directement par gravité dans la goulotte d'alimentation (4c) du secheur vibré (4b) ; en effet la granulation interne au sécheur a couche mince et la siccité des boues préséchées, au-delà de la phase plastique des boues, confèrent à celles-ci une structure optimale pour leur séchage final dans un sécheur vibré à lit fluidisé, sans la nécessité d'avoir un mélangeur granulateur des boues préséchées avec des boues séchées recyclées, entre les deux appareils.
   Le même sécheur vibré (4b) comprend à son extrémité une zone cloisonnée à travers laquelle circule de l'air frais, au moyen des ventilateurs (4e) et (4f) servant au refroidissement des boues séchées à 92 % MS jusqu'à une température inférieure à 50 °C, compatible avec son stockage ultérieur.
   Le sécheur/refroidisseur vibré (4b) ne comporte pas de pales rotatives qui meulent les boues produisant des poussières fines de boues séchées ; les granulés de boue précédemment formés dans le sécheur à couche mince ne sont donc pas détruits dans le sécheur/refroidisseur vibré, seule leur grosseur est légèrement réduite du fait de la réduction de leur teneur en eau. Il en résulte l'absence de poussières dans le produit sec final et dans les gaz chauds d'échappement ; un cyclone (4g) est toutefois installé sur le circuit de sortie des gaz chauds à titre de sécurité.
   Les boues séchées refroidies sont amenées par un transporteur à bande (4h) avec une couverture translucide, et un élévateur à godets (4i) dans un silo de stockage (4j) équipé d'une goulotte télescopique (4k), avant leur transport par camion muni d'un système pneumatique de déchargement des boues séchées sur le site de l'usine d'incinération des ordures ménagères la plus proche.
   Selon des modes particuliers de réalisation, le cyclone de sécurité peut être soit supprimé soit remplacé par tout autre dispositif tel que filtre à manches.
5 ) La condensation des buées issues du sécheur indirect à couche mince, (5), avec récupération de la chaleur de condensation à l'aide du condenseur tubulaire (5a). Cette récupération d'énergie des buées sous forme d'eau chaude, sert au chauffage des boues fraîches liquides avant leur digestion anaérobie, et au chauffage des locaux de la station d'épuration. Le condenseur par mélange (5b) sert par injection d'eau épurée, à condenser un excédent éventuel de buées et est dimensionné pour condenser, le cas échéant, la totalité des buées issues du sécheur à couche mince.
   Les composés volatils contenus dans les boues, tels que : mercaptans, formaldéhyde et hydrogène sulfuré, se retrouvent entraînés dans les buées issues du sécheur indirect à couche mince où les boues sont préséchées jusqu'à environ 64 % MS ; à ce stade tous les composés volatils malodorants se trouvent évaporés et entraînés par les buées vers les condenseurs ; les gaz incondensables sortie condenseurs représentent un faible débit et sont aspirés à travers un séparateur de gouttelettes (5c), par un ventilateur (5d) et refoulés vers le foyer de la turbine à gaz pour y être brûlés et ainsi désodorisés thermiquement ; ainsi les boues préséchées à 64 % MS se trouvent débarrassées des composés volatils malodorants et peuvent être séchées dans le sécheur vibré à lit fluidisé à l'aide des gaz chauds sans le risque de transmettre à ces derniers une pollution olfactive importante, de telle sorte que la désodorisation des gaz chauds sortie sécheur/ refroidisseur vibré n'est pas nécessaire, ce qui simplifie considérablement le procédé selon l'invention.
   Selon des modes particuliers de réalisation, dans le procédé selon l'invention, la désodorisation des gaz incondensables sortie condenseurs, peut être réalisée par voie chimique ou biologique, et le sécheur/refroidisseur vibré peut être équipé dans le procédé selon l'invention, d'une installation pour la désodorisation des gaz chauds d'échappement.
6 ) La valorisation énergétique « optimisée» des boues séchées à 92 % MS environ, objet de la revendication 3, est caractérisée en ce que :
   - Les boues séchées représentant un faible volume, sont transportées par camion sur le site de l'usine d'incinération des ordures ménagères la plus proche où il sera installé, dans le cadre du procédé de l'invention, un silo de stockage adéquat (6a) des boues séchées ainsi qu'un équipement de dosage (6b) automatique du débit des boues séchées.
   - Les boues séchées dont le PCI est égal à environ 2270 kcal/kg ou 2,64 kwh/kg, sont dosées automatiquement dans chaque four-chaudière (7) en fonction du débit vapeur de consigne, et compenseront les creux des oscillations de la charge thermique des ordures ménagères, jusqu'à la ligne horizontale correspondant à la charge thermique nominale du four-chaudière, sans que cela nécessite un surdimensionnement des équipements existants de l'usine d'incinération des ordures ménagères. Ce mode de valorisation énergétique des boues séchées présente l'avantage pour l'usine d'incinération des ordures ménagères, de disposer d'un combustible d'appoint aisément stockable et dosable, pour produire un débit vapeur « constant » et pouvoir assurer une livraison « garantie » d'énergie à un tiers.

Une usine d'incinération des ordures ménagères, est déclarée apte à brûler les boues séchées, si elle est équipée en plus de la récupération d'énergie, d'installation d'épuration des fumées (8) conforme aux règlements en vigueur en vue de la protection des sols, des nappes et de l'environnement, en particulier concernant l'élimination des métaux lourds des ordures ménagères et des boues séchées. Sachant la relative faible proportion du tonnage des boues séchées par rapport à celui des ordures ménagères, généralement égale à 4,5%, l'élimination des métaux lourds en provenance des boues séchées entraînera un surcoût « marginal », à déterminer et à comparer au cas par cas avec le bénéfice de la valorisation de l'énergie contenue dans les boues séchées.

Selon des modes particuliers de réalisation, la destination des boues séchées peut être dans le procédé selon l'invention, différente de celle exposée ci-dessus telle que toute autre installation d'incinération.

Tout le matériel de liaison, de mesures et régulations, de manutention et stockage des boues etc..., est caractérisé en ce qu'il sera choisi à la pointe de la technique à la date de chaque réalisation du procédé selon l'invention ; les représentations schématiques de ce matériel figurant sur le schéma de procédé annexé à l'exception des sécheurs ne sont pas limitatives.

Le procédé selon l'invention réduit le tonnage des boues d'épuration, au fur et à mesure des étapes de traitement, comme l'indique le tableau ci-dessous :

| Boues d'épuration en T/1000 EH/an : | | | | |
|---|---|---|---|---|
| B.fraîches (4-5 %MS) | B.digérées. (4-5 %MS) | B.déshydratées (22-24 %MS) | B.séchées (92 %MS) | B.incinérées (100% M.min.) |
| 511 | 350,5 | 104,5 | 17,14 | 7,82 |

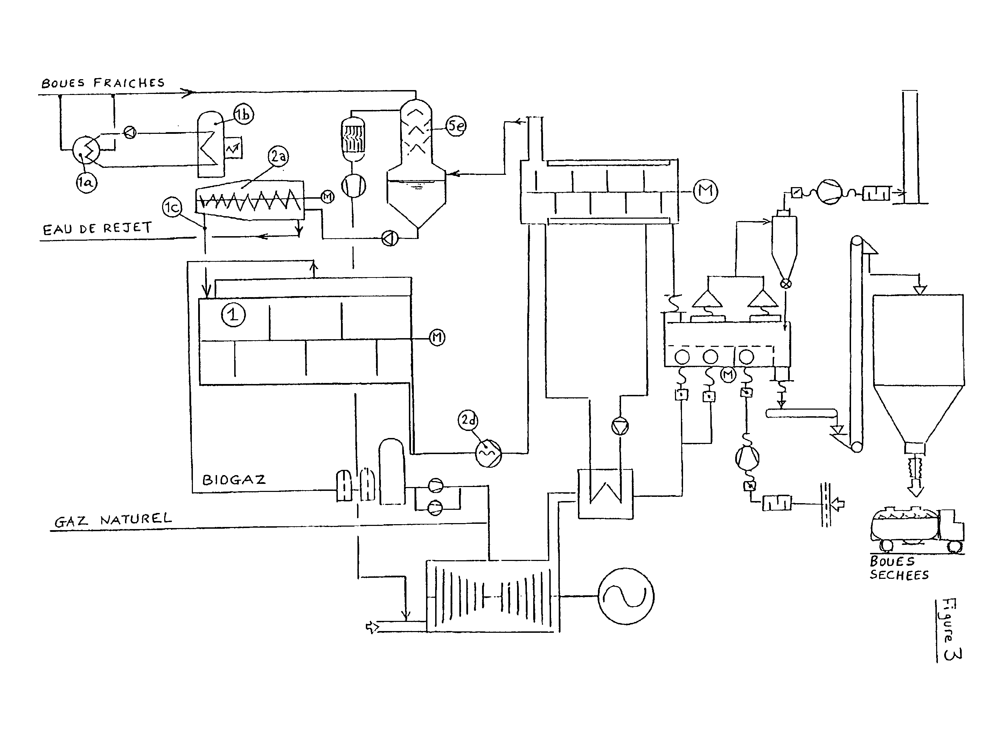

En référence à la figure 3, la « variante », objet de la revendication 4, du procédé objet de l'invention, est caractérisée en ce que les fonctions 3, 4 et 6 du procédé sont inchangées, et que les fonctions 1, 2 et 5 peuvent être réalisées, de façon inédite dans le cadre du procédé, comme suit : les boues fraîches liquides sont chauffées à la température de 55-60 °C dans le condenseur (5e) des buées issues du sécheur à couche mince, puis déshydratées en continu par centrifugation (2a) avec adjonction préalable de polymères ; la quantité de polymères utilisée est plus faible et la siccité des boues fraîches déshydratées est plus élevée, du fait de l'élévation de la température des boues à 55-60 °C. Pour le premier démarrage du chauffage des boues fraîches seront utilisés la chaudière électrique (1b) et l'échangeur de chaleur (1a) qui, en cas de besoin, serviraient d'appoint.

Les boues déshydratées à 28-30 % de matières sèches, alimentent directement par gravité à travers une goulotte (1c), le digesteur anaérobie thermophile horizontal (1), muni d'un agitateur assurant le mélange des boues au cours de leur digestion et un dégazage optimal ; le volume du digesteur est beaucoup plus réduit du fait de l'élévation de la siccité des boues fraîches entrantes à 28-30 % MS au lieu de 4-5 % MS, entraînant une réduction du volume du digesteur d'environ 6 fois pour une durée de séjour inchangée.

Les boues digérées sont extraites, en continu, à l'extrémité du réacteur horizontal, et sont dosées à l'aide d'une pompe spéciale (2d) directement, sans silo de stockage intermédiaire, vers le séchage thermique des boues digérées.

Le digesteur horizontal est dimensionné avec une réserve de volume suffisante pour pallier à un arrêt intempestif du séchage thermique des boues. La durée du séjour des boues dans le réacteur est voisine de 20 jours. Les fonctions 3, 4 et 6 du procédé sont ensuite réalisées sans changement.

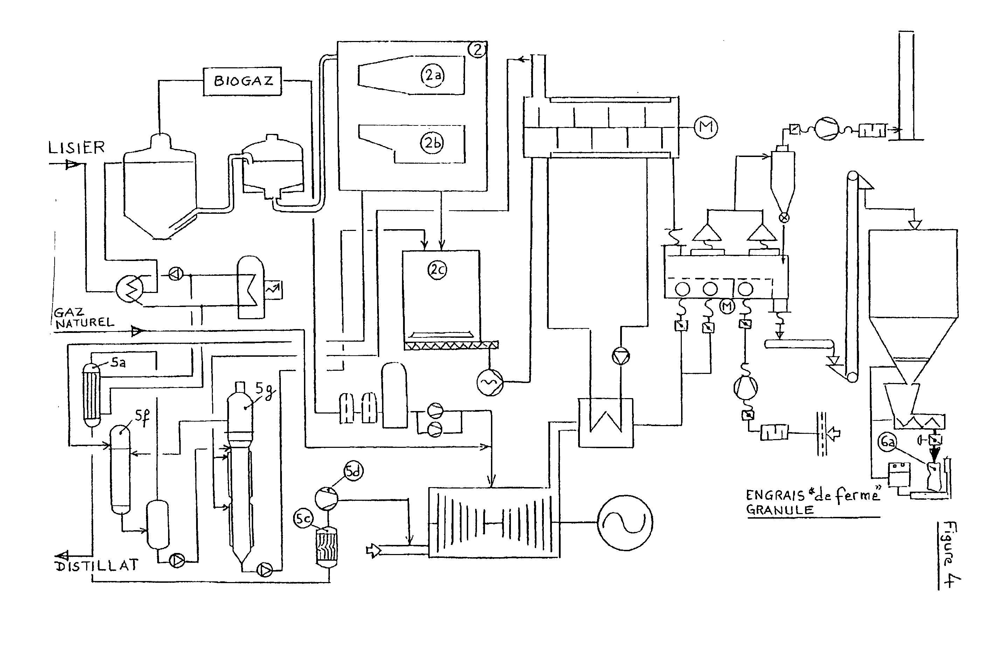

En référence à la figure 4, l'« application », objet de la revendication 5, du procédé objet de l'invention, au traitement du « lisier », est caractérisée en ce que les fonctions 1, 2, 3 et 4 du procédé sont inchangées, et que les fonctions 5 et 6 peuvent être réalisées, de façon inédite dans le cadre du procédé, comme suit :

La condensation des buées issues du sécheur indirect à couche mince s'effectue d'une part dans le condenseur tubulaire (5a), produisant l'eau chaude pour le chauffage du digesteur anaérobie, et d'autre part dans un évaporateur à couche mince (5g) avec un rotor muni de palettes mobiles qui, en cours de marche, s'appliquent sur la paroi chauffée en vue de finir la concentration à environ 20% de matières sèches, du « filtrat » provenant de la déshydratation mécanique du lisier ; la préconcentration du filtrat du lisier est réalisée dans une installation à multiple effet (5f) d'évaporateurs à film tombant ; le concentrat à environ 20% MS est amené dans le silo de stockage tampon (2c) ; le distillat, constitué d'eau épurée, est utilisée pour l'irrigation ou rejetée dans le milieu naturel. Les gaz incondensables sortie condenseurs représentent un faible débit et sont aspirés à travers un séparateur de gouttelettes (5c), par un ventilateur (5d) et refoulés vers le foyer de la turbine à gaz pour y être brûlés et ainsi désodorisés thermiquement.

Le lisier séché est valorisé comme un engrais « de ferme » granulé, ensaché à l'aide de l'ensacheuse (6a) ; il est riche en éléments nutritifs et sera en priorité utilisé en fonction des besoins des plantes, sans surdosage, au même titre qu'un engrais du commerce.

## Revendications

1. Procédé de traitement et de valorisation énergétique des boues d'épuration d'origine urbaine et industrielle, permettant par la juxtaposition inédite d'équipements connus et éprouvés, l'obtention d'un bilan énergétique positif égal à ≅ 5,7 tonnes équivalent pétrole par an et par 1000 équivalents habitants; il est **caractérisé en ce qu'**il comporte les étapes suivantes au nombre de six :
◆ la digestion anaérobie des boues (1), à une phase, à deux phases ou précédée d'une stabilisation aérobie thermophile, et production de biogaz,
◆ la déshydratation mécanique (2) des boues digérées,
◆ la combustion du biogaz, enrichi avec un faible appoint de gaz naturel, dans une turbine à gaz (3) fonctionnant « en énergie totale »,
◆ le séchage thermique des boues déshydratées avec deux appareils en série : le premier appareil est un sécheur à couche mince (4a) à double enveloppe, chauffée par l'huile thermique en circulation (3b) dans la chaudière de récupération (3a) de la chaleur des gaz de combustion du biogaz, le deuxième appareil est un sécheur/ refroidisseur (4b) vibré à lit fluidisé, chauffé par les gaz chauds (3c) sortie chaudière de récupération et muni à sa sortie gaz d'un cyclone (4g), à titre de sécurité,
◆ la condensation des buées issues du sécheur indirect à couche mince, avec désodorisation thermique des gaz incondensables dans le foyer de la turbine à gaz, dans un condenseur tubulaire (5a) assurant l'eau chaude nécessaire au chauffage du digesteur et des locaux, et en secours dans un condenseur par mélange (5b) par injection d'eau épurée,
◆ le transport des boues séchées sur le site d'une usine d'incinération des ordures ménagères la plus proche, et leur « valorisation » énergétique comme combustible d'appoint stockable (6a) et automatiquement dosé (6b) en fonction de la demande des fours-chaudières existants (7) de manière à compenser les creux de la charge thermique et à utiliser au mieux la capacité des équipements existants de l'usine d'incinération, y compris ceux relatifs à l'élimination des métaux lourds (8), « sans » nécessiter leur surdimensionnement, imputable au traitement des boues.

2. Procédé selon la revendication 1 **caractérisé en ce que** l'énergie générée par la combustion du biogaz dans l'installation de cogénération, étape 3 du procédé selon la revendication 1, est « totalement » utilisée, aux pertes par déperditions près, pour les besoins du séchage thermique des boues d'épuration ou du lisier, avec un rendement global énergétique supérieur de 16 % à celui d'une installation classique de cogénération ; et ce grâce à l'emploi judicieux de deux sécheurs appropriés : le premier sécheur (4a), du type à couche mince, chauffé indirectement par l'huile thermique (3b) réchauffée dans la chaudière de récupération (3a), et le deuxième sécheur (4b), du type vibré à lit fluidisé, chauffé directement par les gaz de combustion du biogaz sortie chaudière (3c).

3. Procédé selon la revendication 1 **caractérisé en ce que** les boues séchées dont le pouvoir calorifique inférieur (PCI) est égal à environ 2270 Kcal/Kg, sont utilisées comme combustible pauvre par l'usine, la plus proche, d'incinération des ordures ménagères avec récupération d'énergie et élimination des métaux lourds ; ce qui lui permet grâce à cet apport de combustible d'appoint aisément stockable et « dosable », d'assurer une livraison constante et garantie de l'énergie récupérée.

4. Procédé selon la revendication 1 **caractérisée en ce que** les boues fraîches liquides sont d'abord déshydratées mécaniquement, par centrifugation continue (2a), puis digérées en anaérobie thermophile dans un réacteur horizontal (1) ; le chauffage des boues fraîches à la température de 55-60 °C utilisant la chaleur des buées issues du sécheur à couche mince (4a), est réalisé avec des boues liquides dans un condenseur par mélange (5e) muni de cascades, ce qui améliore les performances de la centrifugation qui fournit des boues déshydratées à 28-30 % de matières sèches, celles-ci alimentant directement le digesteur à la température voulue, et progressent à l'intérieur par un effet de « piston » jusqu'à l'extrémité, puis sont dosées par une pompe (2d) vers le sécheur à couche mince (4a), les fonctions 3, 4 et 6 du procédé objet de la revendication 1, étant alors utilisées sans changement.

5. Procédé selon la revendication 1 **caractérisé en ce que**, pour le traitement et transformation en engrais granulé du « lisier », sont apportées les adaptations suivantes :
- le filtrat de la déshydratation mécanique est préconcentré dans uns installation à multiple effet (5f) d'évaporateurs à film tombant, puis concentré jusqu'à environ 20 % de matières sèches dans un évaporateur à couche mince (5g) avec un rotor muni de palettes mobiles, au moyen de l'énergie fournie par les buées excédentaires par rapport au besoin de chauffage du digesteur.
- le lisier séché est ici valorisé comme un engrais « de ferme » granulé ; après séchage/ refroidissement, il est stocké puis ensaché à l'aide de l'ensacheuse (6a).

6. Procédé selon la revendication 1 **caractérisé en ce que** la turbine à gaz (3) est remplacée par un moteur à gaz ou tout autre système de cogénération.

7. Procédé selon la revendication 1 **caractérisé en ce que** le cyclone (4g) est soit supprimé, soit remplacé par tout autre dispositif tel que filtre à manches.

8. Procédé selon la revendication 1 **caractérisé en ce que** la désodorisation des gaz incondensables ou des gaz d'échappement est réalisée par voie chimique ou biologique.

9. Procédé selon la revendication 1 **caractérisé en ce que** les boues séchées ne sont pas employées comme combustible d'appoint dans une usine d'incinération d'ordures ménagères, mais dans toute autre installation d'incinération.

## Patentansprüche

1. Verfahren zur Behandlung und energielieferten Verwertung von kommunalem und industriellem Klärschlamm, das durch ein neuartiges Zusammensetzen von bekannten und erprobten Einrichtungen eine positive Energie Bilanz gleich ≅ 5,7 Tonnen-Petrol-Gleichwert pro Jahr pro 1000 Einwohner-Gleichwert erreicht, **dadurch gekennzeichnet, daß** das Verfahren die sechs folgenden Verfahrensstufen umfaßt :
a) die anaerobe Klärschlammfaulung (1) mit Biogaserzeugung einstufig mit oder ohne einer aeroben thermophilen vorangestellten Stabilisierung oder zweistufig,
b) die mechanische Entwässerung (2) des gefaulten Klärschlammes,
c) die Verbrennung des Biogases in einer Gasturbine (3) nach einer leichten Anreicherung mit Erdgas und mit einer sehr weitgehenden Nutzung der eingesetzen Energie,
d) die thermische Trocknung des entwässerten Klärschlammes mittels zwei Geräte, die serienweise angeordnet sind, **dadurch gekennzeichnet, daß** das erste Gerät ein Dünnschichttrockner (4a) ist mit einem durch Wärmeträgeröl (Kreislauf 3b) beheizten Doppelmantel, dessen Beheizung durch den Wärmetauscher (3a) erfolgt, der die benötigte Wärme aus dem heißen Abgas nach der Biogasverbrennung zurückgewinnt, und daß das zweite Gerät ein vibrierender Fließbetttrockner/ kühler (4b) ist, dessen Beheizung mit dem restlichen heißen Abgas (Kreislauf 3c) nach dem Wärmetauscher erfolgt, und daß das Abgas nach dem Schwingtrockner/kühler (4b) durch einen Zyklon (4g) als Sicherheitsmaßnahme abgesaugt wird,
e) die Brüdenkondensation aus dem indirekten Dünnschichttrockner in einem Rohrkondensator (5a), der das Warmwasser für die Heizung der Faultürme und der Betriebsgebäude erzeugt, oder im Notfall in einem Mischkondensator (5b) durch Einspritzung von gereigneten Abwasser, **dadurch gekennzeichnet, daß** die verbleibende Brüdenabluft nach dem Kondensator der Gasturbine zugeführt wird und in deren Flamme thermisch desodoriert wird,
f) der Transport des getrockneten Klärschlammes auf eine nahe Müllverbrennunganlage und seine energielieferte Verwertung als speichbarer (6a) und automatisch dosierbarer (6b) Zusatzbrennstoff, der den Bedarf der bestehenden Verbrennungsöfen (7) entspricht, damit die üblichen Schwankungen der thermischen Leistung des Müllverbrennungsofens kompensiert werden ohne wegen der Klärschlammbehandlung erforderliche Überdimensionierung der existierenden Einrichtungen inklusive die Abgasereinigungs- und Schwermetallen-Entsorgungsanlage (8).

2. Verfahren nach Anspruch 1, **dadurch gekennzeichnet, daß** die Wärme aus der Verbrennung des Biogases im BHKW (Blockheizkraftwerk) gemäß der dritten Stufe des erfindungssgemäßen Verfahrens für die thermische Klärschlamm- oder Gülletrocknung abgesehen von kleinen Verlusten fast vollständig verwendet wird, daß der energetische Gesamtwirkungsgrad dank einer gescheiten Anordnung von zwei geeigneten Trocknern 16% höher als der Wirkungsgrad einer herkömmlichen BHKW ist, daß der erste Trockner (4a) ein Dünnschichttrockner ist und mit im Wärmetauscher (3a) erwärmten Wärmeträgeröl (Kreislauf 3b) indirekt geheizt wird, und daß der zweite Trockner (4b) ein vibrierender Fließbetttrockner ist und mit dem restlichen heißen Abgas (Kreislauf 3c) nach dem Wärmetauscher (3a) direkt geheizt wird.

3. Verfahren nach Anspruch 1, **dadurch gekennzeichnet, daß** der getrocknete Klärschlamm mit einem unteren Heizwert (Hu) von ungefähr 2270 kcal/kg (2,64 kWh/kg) als schwacher Brennstoff von einer nahen Müllverbrennungsanlage mit Wärmerückgewinnung und Entsorgung der Schwermetalle benutzt wird, und daß die Verbrennungsanlage dank dieses einfach speichbaren und dosierbaren Zusatzbrennstoffes eine konstante Energielieferung einem Dritte gewährleisten kann.

4. Verfahren nach Anspruch 1, **dadurch gekennzeichnet, daß** der frische flüssige Klärschlamm zuerst mechanisch durch eine Zentrifuge (2a) kontinuierlich entwässert, dann in einem liegenden thermophilen anaeroben Fermenter (1) gefault wird, und daß die für die Faulung benötigte Wärme aus dem Dünnschichttrockner (4a) entweichenden Brüden entzogen wird, wobei der frische flüssige Klärschlamm bis zu einer Temperatur von 55-60°C in einem mit Kaskaden ausgerüsteten Mischkondensator (5a) erwärmt wird, dadurch der entwässerte Klärschlamm nach der Zentrifuge einen höheren TS-Gehalt von 28-30% TS erreicht und direkt den Fermenter in Form einer Pfropfenströmung bis zum Austrag durchläuft, anschließend wird der gefaulte Klärschlamm mittels einer Pumpe (2d) in den Dünnschichttrockner (4a) beschickt, danach werden die Stufen 3, 4, und 6 des Verfahrens nach Anspruch 1 ohne Veränderung durchgeführt.

5. Verfahren nach Anspruch 1, **dadurch gekennzeichnet, daß** die nachfolgenden Veränderungen für die Behandlung und die Veredelung der Gülle zu einem granulierten Hofdünger durchgeführt werden :
- das Filtrat aus der mechanischen Entwässerung wird in einer mehrstufigen Verdampfungsanlage (5f) mit Fallstromverdampfer vorkonzentriert und wird dann bis zu ungefähr 20% TS konzentriert in einem Dünnschichtverdampfer (5g), **dadurch gekennzeichnet, daß** dessen Rotor mit mobilen Schaufeln versehen wird, und dessen Heizung mittels der Kondensationswärme vom Brüdenüberschuß im Vergleich mit für den Fermenter benötigter Wärme erfolgt,
- die getrocknete Gülle wird in diesem Fall als granulierten Hofdünger verwendet, nachdem dieser Dünger gekühlt, gespeichert und mittels einer Big-Bag Befüllungsanlage (6a) verpackt wird.

6. Verfahren nach Anspruch 1, **dadurch gekennzeichnet, daß** die Gasturbine (3) durch einen Gasmotor oder jedes anderes BHKW (Blockheizkraftwerk) ersetzt wird.

7. Verfahren nach Anspruch 1, **dadurch gekennzeichnet, daß** der Zyklon (4g) entweder wegfällt oder durch jede andere geeignete Einrichtung wie einen Schlauchfilter ersetzt wird.

8. Verfahren nach Anspruch 1, **dadurch gekennzeichnet, daß** die verbleibende Brüdenabluft nach dem Kondensator und das Abgas aus der kompletten Anlage chemisch oder biologisch desodoriert werden.

9. Verfahren nach Anspruch 1, **dadurch gekennzeichnet, daß** der getrocknete Klärschlamm nicht in einer Müllverbrennungsanlage sondern in jeder anderen Verbrennungsanlage als Zusatzbrennstoff verwendet wird.

## Claims

1. A process for the treatment and the reuse as solid fuel of urban and industrial sewage sludge, allowing thanks to the novel juxtaposition of known and tested equipment, to obtain a positive net energy balance equal to ≅ 5.7 tons oil equivalent per year per 1000 equivalent inhabitants; it is **characterised that** it comprises the following steps to the number of six :
a) the anaerobic sludge digestion (1) and biogas production, in one stage with or without an aerobic and thermophilic stabilisation, or in two stages,
b) the mechanical dehydration (2) of the digested sludge,
c) the biogas combustion, enriched with a light additional natural gas, in a gas turbine (3) reaching a very high energy global yield thanks to the use of the almost total supplied energy,
d) the thermal drying of the dehydrated sludge by means of two apparatuses in series : the first apparatus is an indirect thin-layer dryer (4a) with a double-mantel heated by a circulating thermal oil (3b) through the boiler (3a) recovering the hot gas heat issued from the biogas combustion, the second apparatus is a vibrated fluid bed dryer and cooler (in its last section) (4b), heated by the exhaust hot gas (3c) out of the recovery heat exchanger and followed by a cyclone separator (4g) in the exhaust gas circuit,
e) the condensation of the vapours coming out of the indirect thin-layer dryer, with a thermally deodorisation of the uncondensable gases in the burner of the gas turbine, by means of a tubular condenser (5a) producing the hot water needed to warm the digester and the premises, and a mix-condenser (5b), as stand-by, to condense the excess vapours in summer time by means of a spray of purified water issued from the sewage treatment plant,
f) the dried sludge transportation to the site of the nearest household refuse incineration plant, and its use as a "booster" fuel which is easily storable in a silo (6a) and automatically conveyed (6b), according to the demand of the existing boiler-furnaces (7), in order to fill or to equalise their thermal load and to utilise at best the existing capacity of the incinerators' equipment, including that related to the heavy metals elimination (8), without any extra cost regarding to dried sludge admission because no extra material capacity of the incinerators' equipment is required.

2. The process according to claim 1, wherein the generated energy by the biogas combustion in the heat-power-coupling unit, corresponding to the 3^{rd} step of the process subject of the invention, is completely used, with deduction of the thermal losses indeed, for the thermal drying needs of either the sewage sludge or the manure, with a total energetic output 16 % higher than that of a conventional unit; this is possible thanks to the following two adequate dryers : the first one (4a), a thin-layer dryer, is indirectly heated by the thermal oil (3b) produced by the recovery boiler (3a), and the second one, a vibrated fluid bed dryer, is directly heated by the boiler exhaust gas (3c) issued from the biogas combustion.

3. The process according to claim 1, wherein the dried sludge the LHV (lower heating value) of which amounts to about 2270 Kcal/Kg, is used as low-energy fuel by the nearest household refuse incineration plant fitted with the heat recovery system and the heavy metals elimination unit; with the advantage for the household refuse incineration plant to benefit from this booster fuel which is easy to store and to feed, for supplying a constant recovered energy.

4. The process according to claim 1, wherein a variant of the process subject of the invention is carried out by inverting the functions 1 and 2 : in fact, the liquid fresh sludge is firstly mechanically dehydrated, by a continuous centrifuge (2a), then thermophilic anaerobic digested in a horizontal reactor (1); the fresh sludge heating up to the temperature of 55-60 °C uses directly the vapours latent heat, issued from the thin-layer dryer (4a), in a mix-condenser (5e) fitted with cascades (no more tubular condenser is used, leading to a simplification of the function 5); this fresh sludge high temperature improves the centrifuge performance, giving a dehydrated sludge at 28-30 % DS, that is directly fed into the digester at the desired temperature and moves forward thanks to a "piston effect" till the extremity, then pumped (2d) to the thin-layer dryer (4a); the functions 3, 4 and 6 of the process subject of the invention are then carried out as specified in claim 1.

5. The process according to claim 1, wherein for the treatment of the livestock manure and its transformation into granulated fertiliser, further comprising the steps of :
Distillation of the liquid issued from the manure mechanical dehydration; which is at first pre-concentrated by means of a multistage evaporation unit (5f) with falling film evaporators, then concentrated till about 20 % dry solid by means of a thin-film evaporator (5g) fitted with moving rotor blades, using the supplied energy from the excess vapours compared with the digester heating need; and
Use of the dried manure, after its cooling and storage, as a granulated "farming" fertiliser, which is bagged by means of the sacking machine (6a).

6. The process according to claim 1, wherein the gas turbine (3) is replaced by a gas motor or any other heat-power-coupling system.

7. The process according to claim 1, wherein the cyclone separator (4g) is either deleted or replaced by any other device such as a bag filter.

8. The process according to claim 1, wherein the deodorisation of the uncondensable gases or the exhaust gas, is carried out either by a chemical or a biological way.

9. The process according to claim 1, wherein the dried sludge is not used as a booster fuel by a household refuse incineration plant, but by any other incineration plant.
